# EUROPEAN PATENT SPECIFICATION

(11) **EP 2 989 112 B1**
(45) Date of publication and mention of the grant of the patent: **22.09.2021**
(21) Application number: 14788130.4
(22) Date of filing: 28.04.2014
(51) Int. Cl.: C07H 19/16, C07H 21/00, A61K 31/7115, C12Q 1/6816, B82Y 5/00

(54) **MODIFIED G-QUADRUPLEX NANOPARTICLES**
MODIFIZIERTE G-QUADRUPLEX-NANOPARTIKEL
NANOPARTICULES G-QUADRUPLEXES MODIFIÉES

(30) Priority: 26.04.2013 US 201361816354 P
(43) Date of publication of application: 02.03.2016
(73) Proprietor: Nanyang Technological University, Singapore 639798 (SG)
(72) Inventor: PHAN, Anh Tuan, Singapore 639798 (SG); LECH, Christopher Brian Henry Jacques, Singapore 639798 (SG)
(74) Representative: Viering, Jentschura & Partner mbB Patent- und Rechtsanwälte
(86) International application number: PCT/SG2014/000189
(87) International publication number: WO 2014/175836

(56) References cited:
- WO-A2-2007/044851
- WO-A2-2008/021193
- CN-A- 101 942 000
- G-X HE ET AL: "N2- and C8-Substituted Oligodeoxynucleotides with Enhanced Thrombin Inhibitory Activity in Vitro and in Vivo", JOURNAL OF MEDICINAL CHEMISTRY, AMERICAN CHEMICAL SOCIETY, US, vol. 41, no. 13, 18 June 1998 (1998-06-18) , pages 2234-2242, XP002454913, ISSN: 0022-2623, DOI: 10.1021/JM970434D
- KOIZUMI M ET AL: "Biologically active oligodeoxyribonucleotides. Part 12:<1> N<2>-Methylation of 2'-deoxyguanosines enhances stability of parallel G-quadruplex and anti-HIV-1 activity", BIOORGANIC & MEDICINAL CHEMISTRY LETTERS, PERGAMON, AMSTERDAM, NL, vol. 10, no. 19, 2 October 2000 (2000-10-02), pages 2213-2216, XP004212006, ISSN: 0960-894X, DOI: 10.1016/S0960-894X(00)00432-7
- H E, G.-X. ET AL.: 'N2-AND C8-SUBSTITUTED OLIGODEOXYNUCLEOTIDES WITH ENHANCED THROMBIN INHIBITORY ACTIVITY IN VITRO AND IN VIVO' JOURNAL OF MEDICINAL CHEMISTRY vol. 41, 1998, pages 2234 - 2242, XP002454913
- KOIZUMI, M. ET AL.: 'Biologically Active Oligodeoxyribonucleotides. Part 12:N2- Methylation of 2'-Deoxyguanosines Enhances Stability of Parallel G-Quadruplex and Anti-HIV-1 Activity' BIOORGANIC & MEDICINAL CHEMISTRY LETTERS vol. 10, 2000, pages 2213 - 2216, XP004212006
- None

### SEQUENCE LISTING

<110> P
   Nanyang Technological university
<120> Modified G-quadruplex nanoparticles
<130> P107882
<160> 49
<170> PatentIn version 3.5
<210> 1
   <211> 24
   <212> DNA
   <213> Artificial Sequence
<220>
   <223> unmodified nucleotide
<400> 1
   ttgggttagg gttagggtta ggga 24
<210> 2
   <211> 24
   <212> DNA
   <213> Artificial Sequence
<220>
   <223> nucleotide with N2-methyl-guanine at position 3
<220>
   <221> misc_feature
   <222> (3)..(3)
   <223> n is N2-methyl-guanine
<400> 2
   ttnggttagg gttagggtta ggga 24
<210> 3
   <211> 24
   <212> DNA
   <213> Artificial Sequence
<220>
   <223> nucleotide with N2-methyl-guanine at position 4
<220>
   <221> misc_feature
   <222> (4)..(4)
   <223> n is N2-methyl-guanine
<400> 3
   ttgngttagg gttagggtta ggga 24
<210> 4
   <211> 24
   <212> DNA
   <213> Artificial Sequence
<220>
   <223> nucleotide with N2-methyl-guanine at position 5
<220>
   <221> misc_feature
   <222> (5)..(5)
   <223> n is N2-methyl-guanine
<400> 4
   ttggnttagg gttagggtta ggga 24
<210> 5
   <211> 24
   <212> DNA
   <213> Artificial sequence
<220>
   <223> nucleotide with N2-methyl-guanine at position 9
<220>
   <221> misc_feature
   <222> (9)..(9)
   <223> n is N2-methyl-guanine
<400> 5
   ttgggttang gttagggtta ggga 24
<210> 6
   <211> 24
   <212> DNA
   <213> Artificial sequence
<220>
   <223> nucleotide with N2-methyl-guanine at position 10
<220>
   <221> misc_feature
   <222> (10)..(10)
   <223> n is N2-methyl-guanine
<400> 6
   ttgggttagn gttagggtta ggga 24
<210> 7
   <211> 24
   <212> DNA
   <213> Artificial Sequence
<220>
   <223> nucleotide with N2-methyl-guanine at position 11
<220>
   <221> misc_feature
   <222> (11)..(11)
   <223> n is N2-methyl-guanine
<400> 7
   ttgggttagg nttagggtta ggga 24
<210> 8
   <211> 24
   <212> DNA
   <213> Artificial sequence
<220>
   <223> nucleotide with N2-methyl-guanine at position 15
<220>
   <221> misc_feature
   <222> (15)..(15)
   <223> n is N2-methyl-guanine
<400> 8
   ttgggttagg gttanggtta ggga 24
<210> 9
   <211> 24
   <212> DNA
   <213> Artificial Sequence
<220>
   <223> nucleotide with N2-methyl-guanine at position 16
<220>
   <221> misc_feature
   <222> (16)..(16)
   <223> n is N2-methyl-guanine
<400> 9
   ttgggttagg gttagngtta ggga 24
<210> 10
   <211> 24
   <212> DNA
   <213> Artificial Sequence
<220>
   <223> nucleotide with N2-methyl-guanine at position 17
<220>
   <221> misc_feature
   <222> (17)..(17)
   <223> n is N2-methyl-guanine
<400> 10
   ttgggttagg gttaggntta ggga 24
<210> 11
   <211> 24
   <212> DNA
   <213> Artificial Sequence
<220>
   <223> nucleotide with N2-methyl-guanine at position 21
<220>
   <221> misc_feature
   <222> (21)..(21)
   <223> n is N2-methyl-guanine
<400> 11
   ttgggttagg gttagggtta ngga 24
<210> 12
   <211> 24
   <212> DNA
   <213> Artificial sequence
<220>
   <223> nucleotide with N2-methyl-guanine at position 22
<220>
   <221> misc_feature
   <222> (22)..(22)
   <223> n is N2-methyl-guanine
<400> 12
   ttgggttagg gttagggtta gnga 24
<210> 13
   <211> 24
   <212> DNA
   <213> Artificial Sequence
<220>
   <223> nucleotide with N2-methyl-guanine at position 23
<220>
   <221> misc_feature
   <222> (23)..(23)
   <223> n is N2-methyl-guanine
<400> 13
   ttgggttagg gttagggtta ggna 24
<210> 14
   <211> 24
   <212> DNA
   <213> Artificial sequence
<220>
   <223> nucleotide with N2-6-amino-hexyl-guanine at position 3
<220>
   <221> misc_feature
   <222> (3)..(3)
   <223> n is N2-6-amino-hexyl-guanine
<400> 14
   ttnggttagg gttagggtta ggga 24
<210> 15
   <211> 24
   <212> DNA
   <213> Artificial Sequence
<220>
   <223> nucleotide with N2-6-amino-hexyl-guanine at position 4
<220>
   <221> misc_feature
   <222> (4)..(4)
   <223> n is N2-6-amino-hexyl-guanine
<400> 15
   ttgngttagg gttagggtta ggga 24
<210> 16
   <211> 24
   <212> DNA
   <213> Artificial sequence
<220>
   <223> nucleotide with N2-6-amino-hexyl-guanine at position 5
<220>
   <221> misc_feature
   <222> (5)..(5)
   <223> n is N2-6-amino-hexyl-guanine
<400> 16
   ttggnttagg gttagggtta ggga 24
<210> 17
   <211> 24
   <212> DNA
   <213> Artificial sequence
<220>
   <223> nucleotide with N2-6-amino-hexyl-guanine at position 9
<220>
   <221> misc_feature
   <222> (9)..(9)
   <223> n is N2-6-amino-hexyl-guanine
<400> 17
   ttgggttang gttagggtta ggga 24
<210> 18
   <211> 24
   <212> DNA
   <213> Artificial sequence
<220>
   <223> nucleotide with N2-6-amino-hexyl-guanine at position 10
<220>
   <221> misc_feature
   <222> (10)..(10)
   <223> n is N2-6-amino-hexyl-guanine
<400> 18
   ttgggttagn gttagggtta ggga 24
<210> 19
   <211> 24
   <212> DNA
   <213> Artificial Sequence
<220>
   <223> nucleotide with N2-6-amino-hexyl-guanine at position 11
<220>
   <221> misc_feature
   <222> (11)..(11)
   <223> n is N2-6-amino-hexyl-guanine
<400> 19
   ttgggttagg nttagggtta ggga 24
<210> 20
   <211> 24
   <212> DNA
   <213> Artificial Sequence
<220>
   <223> nucleotide with N2-6-amino-hexyl-guanine at position 15
<220>
   <221> misc_feature
   <222> (15)..(15)
   <223> n is N2-6-amino-hexyl-guanine
<400> 20
   ttggattagg gttanggtta ggga 24
<210> 21
   <211> 24
   <212> DNA
   <213> Artificial Sequence
<220>
   <223> nucleotide with N2-6-amino-hexyl-guanine at position 16
<220>
   <221> misc_feature
   <222> (16)..(16)
   <223> n is N2-6-amino-hexyl-guanine
<400> 21
   ttgggttagg gttagngtta ggga 24
<210> 22
   <211> 24
   <212> DNA
   <213> Artificial Sequence
<220>
   <223> nucleotide with N2-6-amino-hexyl-guanine at position 17
<220>
   <221> misc_feature
   <222> (17)..(17)
   <223> n is N2-6-amino-hexyl-guanine
<400> 22
   ttgggttagg gttaggntta ggga 24
<210> 23
   <211> 24
   <212> DNA
   <213> Artificial sequence
<220>
   <223> nucleotide with N2-6-amino-hexyl-guanine at position 21
<220>
   <221> misc_feature
   <222> (21)..(21)
   <223> n is N2-6-amino-hexyl-guanine
<400> 23
   ttgggttagg gttagggtta ngga 24
<210> 24
   <211> 24
   <212> DNA
   <213> Artificial Sequence
<220>
   <223> nucleotide with N2-6-amino-hexyl-guanine at position 22
<220>
   <221> misc_feature
   <222> (22)..(22)
   <223> n is N2-6-amino-hexyl-guanine
<400> 24
   ttgggttagg gttagggtta gnga 24
<210> 25
   <211> 24
   <212> DNA
   <213> Artificial Sequence
<220>
   <223> nucleotide with N2-6-amino-hexyl-guanine at position 23
<220>
   <221> misc_feature
   <222> (23)..(23)
   <223> n is N2-6-amino-hexyl-guanine
<400> 25
   ttgggttagg gttagggtta ggna 24
<210> 26
   <211> 24
   <212> DNA
   <213> Artificial Sequence
<220>
   <223> nucleotide with N2-benzyl-guanine at position 3
<220>
   <221> misc_feature
   <222> (3)..(3)
   <223> n is N2-benzyl-guanine
<400> 26
   ttnggttagg gttagggtta ggga 24
<210> 27
   <211> 24
   <212> DNA
   <213> Artificial Sequence
<220>
   <223> nucleotide with N2-benzyl-guanine at position 4
<220>
   <221> misc_feature
   <222> (4)..(4)
   <223> n is N2-benzyl-guanine
<400> 27
   ttgngttagg gttagggtta ggga 24
<210> 28
   <211> 24
   <212> DNA
   <213> Artificial Sequence
<220>
   <223> nucleotide with N2-benzyl-guanine at position 5
<220>
   <221> misc_feature
   <222> (5)..(5)
   <223> n is N2-benzyl-guanine
<400> 28
   ttggnttagg gttagggtta ggga 24
<210> 29
   <211> 24
   <212> DNA
   <213> Artificial Sequence
<220>
   <223> nucleotide with N2-benzyl-guanine at position 9
<220>
   <221> misc_feature
   <222> (9)..(9)
   <223> n is N2-benzyl-guanine
<400> 29
   ttgggttang gttagggtta ggga 24
<210> 30
   <211> 24
   <212> DNA
   <213> Artificial sequence
<220>
   <223> nucleotide with N2-benzyl-guanine at position 10
<220>
   <221> misc_feature
   <222> (10)..(10)
   <223> n is N2-benzyl-guanine
<400> 30
   ttgggttagn gttagggtta ggga 24
<210> 31
   <211> 24
   <212> DNA
   <213> Artificial Sequence
<220>
   <223> nucleotide with N2-benzyl-guanine at position 11
<220>
   <221> misc_feature
   <222> (11)..(11)
   <223> n is N2-benzyl-guanine
<400> 31
   ttgggttagg nttagggtta ggga 24
<210> 32
   <211> 24
   <212> DNA
   <213> Artificial Sequence
<220>
   <223> nucleotide with N2-benzyl-guanine at position 15
<220>
   <221> misc_feature
   <222> (15)..(15)
   <223> n is N2-benzyl-guanine
<400> 32
   ttgggttagg gttanggtta ggga 24
<210> 33
   <211> 24
   <212> DNA
   <213> Artificial Sequence
<220>
   <223> nucleotide with N2-benzyl-guanine at position 16
<220>
   <221> misc_feature
   <222> (16)..(16)
   <223> n is N2-benzyl-guanine
<400> 33
   ttgggttagg gttagngtta ggga 24
<210> 34
   <211> 24
   <212> DNA
   <213> Artificial Sequence
<220>
   <223> nucleotide with N2-benzyl-guanine at position 17
<220>
   <221> misc_feature
   <222> (17)..(17)
   <223> n is N2-benzyl-guanine
<400> 34
   ttgggttagg gttaggntta ggga 24
<210> 35
   <211> 24
   <212> DNA
   <213> Artificial sequence
<220>
   <223> nucleotide with N2-benzyl-guanine at position 21
<220>
   <221> misc_feature
   <222> (21)..(21)
   <223> n is N2-benzyl-guanine
<400> 35
   ttgggttagg gttagggtta ngga 24
<210> 36
   <211> 24
   <212> DNA
   <213> Artificial Sequence
<220>
   <223> nucleotide with N2-benzyl-guanine at position 22
<220>
   <221> misc_feature
   <222> (22)..(22)
   <223> n is N2-benzyl-guanine
<400> 36
   ttgggttagg gttagggtta gnga 24
<210> 37
   <211> 24
   <212> DNA
   <213> Artificial Sequence
<220>
   <223> nucleotide with N2-benzyl-guanine at position 23
<220>
   <221> misc_feature
   <222> (23)..(23)
   <223> n is N2-benzyl-guanine
<400> 37
   ttgggttagg gttagggtta ggna 24
<210> 38
   <211> 24
   <212> DNA
   <213> Artificial Sequence
<220>
   <223> nucleotide with N2-6-amino-hexyl-guanine at positions 3 and 9
<220>
   <221> misc_feature
   <222> (3)..(3)
   <223> n is N2-6-amino-hexyl-guanine
<220>
   <221> misc_feature
   <222> (9)..(9)
   <223> n is N2-6-amino-hexyl-guanine
<400> 38
   ttnggttang gttagggtta ggga 24
<210> 39
   <211> 24
   <212> DNA
   <213> Artificial Sequence
<220>
   <223> nucleotide with N2-methyl-guanine at positions 10 and 11
<220>
   <221> misc_feature
   <222> (10)..(11)
   <223> n is N2-methyl-guanine
<400> 39
   ttgggttagn nttagggtta ggga 24
<210> 40
   <211> 24
   <212> DNA
   <213> Artificial Sequence
<220>
   <223> nucleotide with N2-6-amino-hexyl-guanine at positions 3 and 9 and with N2-methyl-guanine at positions 10 and 11
<220>
   <221> misc_feature
   <222> (3)..(3)
   <223> n is N2-6-amino-hexyl-guanine
<220>
   <221> misc_feature
   <222> (9)..(9)
   <223> n is N2-6-amino-hexyl-guanine
<220>
   <221> misc_feature
   <222> (10)..(11)
   <223> n is N2-methyl-guanine
<400> 40
   ttnggttann nttagggtta ggga 24
<210> 41
   <211> 24
   <212> DNA
   <213> Artificial Sequence
<220>
   <223> nucleotide with N2-6-amino-hexyl-guanine at positions 3 and 9 and with N2-methyl-guanine at positions 11 and 15
<220>
   <221> misc_feature
   <222> (3)..(3)
   <223> n is N2-6-amino-hexyl-guanine
<220>
   <221> misc_feature
   <222> (9)..(9)
   <223> n is N2-6-amino-hexyl-guanine
<220>
   <221> misc_feature
   <222> (11)..(11)
   <223> n is N2-methyl-guanine
<220>
   <221> misc_feature
   <222> (15)..(15)
   <223> n is N2-methyl-guanine
<400> 41
   ttnggttang nttanggtta ggga 24
<210> 42
   <211> 24
   <212> DNA
   <213> Artificial sequence
<220>
   <223> nucleotide with N2-6-amino-hexyl-guanine at positions 3, 4, 9, 11, 15, 17, 22 and 23
<220>
   <221> misc_feature
   <222> (3)..(4)
   <223> n is N2-6-amino-hexyl-guanine
<220>
   <221> misc_feature
   <222> (9)..(9)
   <223> n is N2-6-amino-hexyl-guanine
<220>
   <221> misc_feature
   <222> (11)..(11)
   <223> n is N2-6-amino-hexyl-guanine
<220>
   <221> misc_feature
   <222> (15)..(15)
   <223> n is N2-6-amino-hexyl-guanine
<220>
   <221> misc_feature
   <222> (17)..(17)
   <223> n is N2-6-amino-hexyl-guanine
<220>
   <221> misc_feature
   <222> (22)..(23)
   <223> n is N2-6-amino-hexyl-guanine
<400> 42
   ttnngttang nttangntta gnna 24
<210> 43
   <211> 24
   <212> DNA
   <213> Artificial Sequence
<220>
   <223> nucleotide with N2-6-amino-hexyl-guanine at position 3, with N2-benzyl-guanine at positions 9, 11, 21 and 23, and with N2-methyl-guanine at positions 15 and 17
<220>
   <221> misc_feature
   <222> (3)..(3)
   <223> n is N2-6-amino-hexyl-guanine
<220>
   <221> misc_feature
   <222> (9)..(9)
   <223> n is N2-benzyl-guanine
<220>
   <221> misc_feature
   <222> (11)..(11)
   <223> n is N2-benzyl-guanine
<220>
   <221> misc_feature
   <222> (15)..(15)
   <223> n is N2-methyl-guanine
<220>
   <221> misc_feature
   <222> (17)..(17)
   <223> n is N2-methyl-guanine
<220>
   <221> misc_feature
   <222> (21)..(21)
   <223> n is N2-benzyl-guanine
<220>
   <221> misc_feature
   <222> (23)..(23)
   <223> n is N2-benzyl-guanine
<400> 43
   ttnggttang nttangntta ngna 24
<210> 44
   <211> 24
   <212> DNA
   <213> Artificial Sequence
<220>
   <223> nucleotide with N2-6-amino-hexyl-guanine at positions 3 and 23, with N2-benzyl-guanine at positions 4, 9, 11 and 21, and with N2-methyl-guanine at positions 10, 15, 17 and 22
<220>
   <221> misc_feature
   <222> (3)..(3)
   <223> n is N2-6-amino-hexyl-guanine
<220>
   <221> misc_feature
   <222> (4)..(4)
   <223> n is N2-benzyl-guanine
<220>
   <221> misc_feature
   <222> (9)..(9)
   <223> n is N2-benzyl-guanine
<220>
   <221> misc_feature
   <222> (10)..(10)
   <223> n is N2-methyl-guanine
<220>
   <221> misc_feature
   <222> (11)..(11)
   <223> n is N2-benzyl-guanine
<220>
   <221> misc_feature
   <222> (15)..(15)
   <223> n is N2-methyl-guanine
<220>
   <221> misc_feature
   <222> (17)..(17)
   <223> n is N2-methyl-guanine
<220>
   <221> misc_feature
   <222> (21)..(21)
   <223> n is N2-benzyl-guanine
<220>
   <221> misc_feature
   <222> (22)..(22)
   <223> n is N2-methyl-guanine
<220>
   <221> misc_feature
   <222> (23)..(23)
   <223> n is N2-6-amino-hexyl-guanine
<400> 44
   ttnngttann nttangntta nnna 24
<210> 45
   <211> 24
   <212> DNA
   <213> Artificial Sequence
<220>
   <223> nucleotide with N2-C-12-Biotin-2'deoxy guanosine-amidite at position 22
<220>
   <221> misc_feature
   <222> (22)..(22)
   <223> n is N2-C-12-Biotin-2'-deoxy guanosine-amidite
<400> 45
   ttgggttagg gttagggtta gnga 24
<210> 46
   <211> 24
   <212> DNA
   <213> Artificial Sequence
<220>
   <223> nucleotide with N2-C-12-Biotin-2'deoxy guanosine-amidite at positions 11 and 22
<220>
   <221> misc_feature
   <222> (11)..(11)
   <223> n is N2-C-12-Biotin-2'deoxy guanosine-amidite
<220>
   <221> misc_feature
   <222> (22)..(22)
   <223> n is N2-C-12-Biotin-2'deoxy guanosine-amidite
<400> 46
   ttgggttagg nttagggtta gnga 24
<210> 47
   <211> 24
   <212> DNA
   <213> Artificial Sequence
<220>
   <223> nucleotide with N2-C-12-Biotin-2'deoxy guanosine-amidite at positions 3 and 23
<220>
   <221> misc_feature
   <222> (3)..(3)
   <223> n is N2-C-12-Biotin-2'deoxy guanosine-amidite
<220>
   <221> misc_feature
   <222> (23)..(23)
   <223> n is N2-C-12-Biotin-2'deoxy guanosine-amidite
<400> 47
   ttnggttagg gttagggtta ggna 24
<210> 48
   <211> 24
   <212> DNA
   <213> Artificial sequence
<220>
   <223> nucleotide with N2-C-12-Biotin-2'deoxy guanosine-amidite at positions 15, 17 and 22
<220>
   <221> misc_feature
   <222> (15)..(15)
   <223> n is N2-C-12-Biotin-2'deoxy guanosine-amidite
<220>
   <221> misc_feature
   <222> (17)..(17)
   <223> n is N2-C-12-Biotin-2'deoxy guanosine-amidite
<220>
   <221> misc_feature
   <222> (22)..(22)
   <223> n is N2-C-12-Biotin-2'deoxy guanosine-amidite
<400> 48
   ttgggttagg gttangntta gnga 24
<210> 49
   <211> 24
   <212> DNA
   <213> Artificial Sequence
<220>
   <223> nucleotide with N2-C-12-Biotin-2'deoxy guanosine-amidite at positions 9, 17 and 22
<220>
   <221> misc_feature
   <222> (9)..(9)
   <223> n is N2-C-12-Biotin-2'deoxy guanosine-amidite
<220>
   <221> misc_feature
   <222> (17)..(17)
   <223> n is N2-C-12-Biotin-2'deoxy guanosine-amidite
<220>
   <221> misc_feature
   <222> (22)..(22)
   <223> n is N2-C-12-Biotin-2'deoxy guanosine-amidite
<400> 49
   ttgggttang gttaggntta gnga 24

## Description

### FIELD OF THE INVENTION

The present invention lies in the field of biochemistry and relates to a nucleic acid nanoparticle comprising at least one G-quadruplex structure wherein at least one guanine base comprises a modification in the N2-position and the use of said nucleic acid nanoparticle as a medicament and to methods of stabilizing a nucleic acid nanoparticle comprising at least one G-quadruplex structure. Further, the present invention relates to the use of nucleic acid molecules comprising at least one guanine base comprising a modification in the N2-position as molecular sensors or as a tag.

### BACKGROUND OF THE INVENTION

G-quadruplex nucleic acids are four-stranded structures formed by certain guanine-rich sequences. These structures have gained research attention based on their various biological functions and their role as anti-cancer drug targets (Patel, DJ et al. (2007) Nucleic Acid Research 35, 22, pages 7429-7455). Many potential drug candidates have been engineered from the G-quadruplex scaffold displaying a wide array of pharmacological activity, for example against cancer, HIV and blood coagulation (Bock, LC et al. (1992) Nature 355, 6360, pages 564-566, de Soultrait, VR et al. (2002) J Mol Biol 324, 2, pages 195-203, Jing, NJ et al. (2004) Cancer Res 64 (18), pages 6603-6609). Besides their pharmaceutical use, other applications of G-quadruplex have been demonstrated, including their use as catalysts, sensing devices, molecular electronics systems, and nano-particle scaffolds (Chinnapen, DJF and Sen, D (2004) P Natl Acad Sci USA 101, 65-69; Wieland, M and Hartig, JS (200&) Angew Chem Int Edit 45, 5875-5878, (2006); Alberti, P et al. (2006) Org Biomol Chem 4, 3383-3391; Juskowiak, B (2006) Anal Chim Acta 568, 171-180, (2006); Tang, Z et al. (2008) Chembiochem 9, 1061-1064; Cai, JF et al. (2009) Chem 20, 205-208; Huang, YC and Sen, DA (2010) J Am Chem Soc 132, 2663-2671).

One reason for the use of G-quadruplexes in the above described technologies is their ability to be functionalized. For example, functionalization of strand termini can enhance the activity of G-quadruplex based aptamers (Koizumi, M et al. (1997) Bioorg Med Chem 5, 12, pages 2235-2243) and enables their use as FRET based sensors (Juskowiak, B (2006) Anal Chim Acta 568, 1-2, pages 171-180). While modification of strand termini is useful, such modifications are restricted by the location and number of strand termini in the G-quadruplex molecule. Another opportunity to modify the G-quadruplex is the substitution of guanine bases located in the core of the G-quadruplex scaffold with chemically altered guanine bases (Sagi, J (2013) J Biomol Struct Dyn). In theory, the modification of a guanine base allows for functionalization at a desired location on the G-quadruplex nanoparticle, and can accommodate up to four modified guanines per tetrad layer.

However, many types of guanine analogs have been observed to destabilize the G-quadruplex structure (Gros, J (2007) Nucleic Acid Res 35, 9, pages 3064-3075). This is especially true for modifications that disrupt the Hoogsteen bonding of the guanine tetrad, such as modification at the O6-position (Mekmaysy, CS (2008) J Am Chem Soc 130, 21, pages 6710-6711). Also, modification at the C8 position of the guanine base has received much research attention (Sagi, J (2013) J Biomol Struct Dyn). Previous studies have shown that modification at the C8-position affects the stability of G-quadruplex formation in a manner dependent on guanine base conformation (Lech, CJ (2011) Biophys J 101, 8, pages 1987-1998). This conformational dependence limits the ability to functionalize at the C8-position, with only substitutions to guanines in a syn-orientation being effective stabilizers. The international patent publications WO 2007/044851 A2 and WO 2008/021193 A2 as well as He et al. (J Med. Chem. (1998) Vol. 41, No. 13, pages 2234-2242) and Koizumi et al. (Bioorganic and Medicinal Chem. Lett. (2000) Vol. 10, No. 19, pages 2213-2216) disclose nucleic acids comprising at least one G quadruplex structure wherein at least one guanine base comprises a modification in the N2 position.

However, there is need for other classes of nucleotide chemistries that allow for the formation and functionalization of G-quadruplex based nanoparticles.

### SUMMARY OF THE INVENTION

It is an object of the present invention to meet the above need by providing a nucleic acid nanoparticle comprising at least one G-quadruplex structure containing more than 2 G-tetrad layers wherein at least two guanine bases comprise a modification in the N2-position that stabilizes the nucleic acid nanoparticle, wherein the G-quadruplex structure comprises (a) a nucleic acid molecule comprising the nucleic acid sequence (g)w(n)a(g)x(n)b(g)y(n)c(g)z wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)z wherein z is an integer of at least 2; or (c) two nucleic acid molecules wherein each of said molecules comprises a sequence of (g)y(n)b(g)z wherein b is an integer of at least 0, y and z are independently of each other integers of at least 0, and the sum of integers y and z is at least 4; and wherein each modification is independently 6-aminohexyl or benzyl. Surprisingly, the inventors have found that such modification stabilizes the nanoparticle and allows the attachment of additional compounds, such as pharmaceutically active compounds or compounds that enable the nanoparticles use as a molecular sensor or tag, to functionalize the nanoparticle. Hence, a nucleic acid nanoparticle comprising at least one G-quadruplex structure wherein at least one guanine base comprises a modification in the N2-position and being functionalized with a pharmaceutically active compound can be used as a medicament. Further, the stabilization effect of the modification at the N2-position provides the basis for methods for stabilizing a nucleic acid nanoparticle comprising at least one G-quadruplex structure.

The versatility of N2-modifications has been demonstrated in a structurally diverse (3+1) G-quadruplex scaffold. The results indicate that single substitutions of N2-modified guanines are potent stabilizers of the G-quadruplex architecture. It has also been demonstrated that multiple N2-modifications can be easily incorporated into the G-quadruplex leading to an incredibly stable scaffold. In theory, such a scaffold could accommodate up to 4 functionalized chemical groups per layer for a total of 12 modified groups in a 3-layered G-quadruplex. Furthermore, it has been experimentally shown that the addition of 8 N2-modified chemical groups to a 3-layered G-quadruplex scaffold yields a remarkable increase in stability of >45°C without disrupting the conformation of the G-quadruplex.

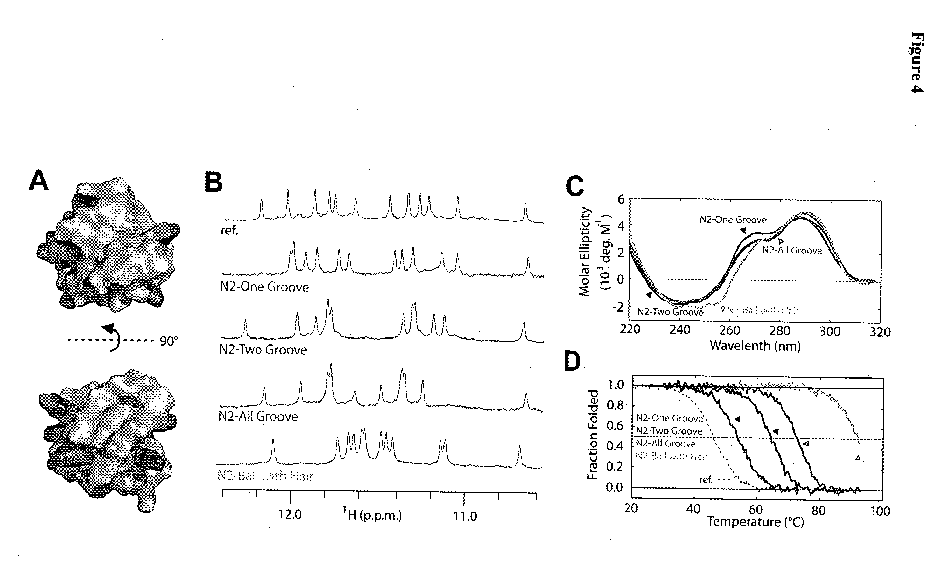

Also the ability to modify the G-quadruplex scaffold with a variety of chemical groups, including long chain-like residues that can be used as a bridge to functionalize the molecular scaffold has been demonstrated. The ability of the G-quadruplex to host multiple N2-modifications without disrupting the structure provides for the possibility of functionalizing this nanoparticle in a ball-with-hair type fashion (Fig. 4A). The results presented in the current application are directly applicable to the design of highly stable G-quadruplex based nanoparticle systems. Further, these results also suggest that N2-modified nucleic acid nanoparticles may represent a new opportunity for stabilizing existing G-quadruplex based drugs.

In a first aspect, the present invention is thus directed to a nucleic acid nanoparticle comprising at least one G-quadruplex structure containing more than 2 G-tetrad layers wherein at least two guanine bases comprise a modification in the N2-position that stabilizes the nucleic acid nanoparticle, wherein the G-quadruplex structure comprises (a) a nucleic acid molecule comprising the nucleic acid sequence (g)w(n)a(g)x(n)b(g)y(n)c(g)z wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)z wherein z is an integer of at least 2; or (c) two nucleic acid molecules wherein each of said molecules comprises a sequence of (g)y(n)b(g)z wherein b is an integer of at least 0, y and z are independently of each other integers of at least 0, and the sum of integers y and z is at least 4; and wherein each modification is independently 6-aminohexyl or benzyl.

In a further aspect, the present invention relates to a nucleic acid nanoparticleas described herein for use as a medicament.

In a still further aspect, the present invention relates to the use of a nucleic acid molecule as a molecular sensor, wherein said nucleic molecule comprises (a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)_{z} wherein z is an integer of at least 2; or (c) two nucleic acid molecules wherein each of said molecules comprises a sequence of (g)_{y}(n)_{b}(g)_{z} wherein b is an integer of at least 0, y and z are independently of each other integers of at least 0, and the sum of integers y and z is at least 4, wherein the nucleic acid molecule comprises at least 2 and preferably up to 10 N2-modified guanine bases, and wherein each modification is independently 6-aminohexyl or benzyl.

The scope of protection is defined by the appended claims.

According to the invention, the G-quadruplex structure comprises (a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)_{z} wherein z is an integer of at least 2; or (c) two nucleic acid molecules wherein each of said molecules comprises a sequence of (g)_{y}(n)_{b}(g)_{z} wherein b is an integer of at least 0, y and z are independently of each other integers of at least 0, and the sum of integers y and z is at least 4.. In various embodiments, the nucleic acid molecule comprises the nucleic acid sequence (g)_{w}(n)ₐ(g)ₓ(n)_{b}(g)_{y}(n)_{c}(g)_{z} wherein w,x,y,z are independently of each other integers of at least 3 and a,b,c are independently of each other integers of at least 1. In certain embodiments, the at least one N2-modified guanine base is located in one of the (g) sequence elements and not in the (n) sequence elements. In other certain embodiments, the G-quadruplex structure comprises a nucleic acid molecule having the nucleic acid sequence (n)₂-(g)₃-(n)₃-(g)₃-(n)₃-(g)₃-(n)₃-(g)₃-(n).

In various embodiments, the G-quadruplex structure comprises at least two N2-modified guanine bases. In various embodiments, the G-quadruplex structure comprises up to four N2-modified guanine bases. In various other embodiments, the G-quadruplex structure comprises up to 10 N2-modified guanine bases. In various other embodiments, all guanine bases are N2-modified.

According to the invention, the modification is independently 6-amino-hexyl or benzyl.

In various embodiments, the modification comprises a pharmaceutically active compound.

In various embodiments, the G-quadruplex structure comprises one nucleic acid molecule. In various other embodiments, the G-quadruplex structure comprises at least two nucleic acid molecules, such as 2, 3, 4, 5, 6, 7, 8, 9, ... nucleic acid molecules.

In various embodiments, the G-quadruplex structure has a diameter of 2 to 3 nm. In certain embodiments, the G-quadruplex structure comprises a nucleic acid molecule having a tag at its 3' and/or 5' end.

In various embodiments, a given guanine base comprises only one modification in its N2-postion. In various embodiments, the nucleic acid nanoparticle comprises only one G-quadruplex structure.

### BRIEF DESCRIPTION OF THE DRAWINGS

The invention will be better understood with reference to the detailed description when considered in conjunction with the non-limiting examples and the accompanying drawings.

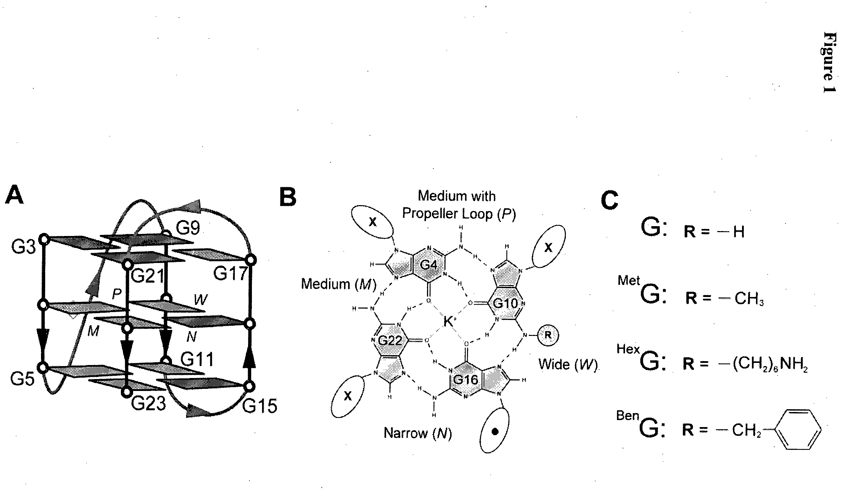

**Figure 1** (A) shows a scheme of the (3+1) hybrid G-quadruplex studied in this in application. Groove widths and select guanine residues are labeled. Guanine bases are colored based on their syn (magenta) or anti (cyan) glycosidic conformation. (B) Example of a single-position N2-modified guanine substitution within the middle G-tetrad layer. Groove descriptions are shown. The 5'→3' strand directionality is indicated as into (x) and out of (•) the page. (C) Description of the N2-guanine modifications used in this application.

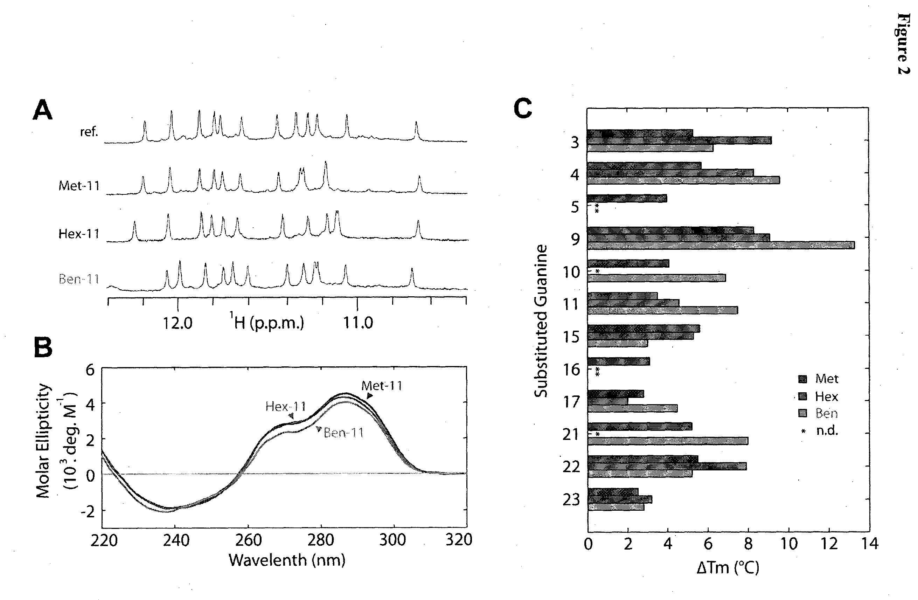

**Figure 2** shows G-quadruplex nanoparticles containing single N2-modifications: (A-B) NMR and CD spectra of representative N2-modified sequences for each of the ^{Met}G, ^{Hex}G, and ^{Ben}G chemistries that maintained the original (3+1) folding topology. (C) Change in melting temperature (Tₘ) observed for single-position N2-modified sequences.

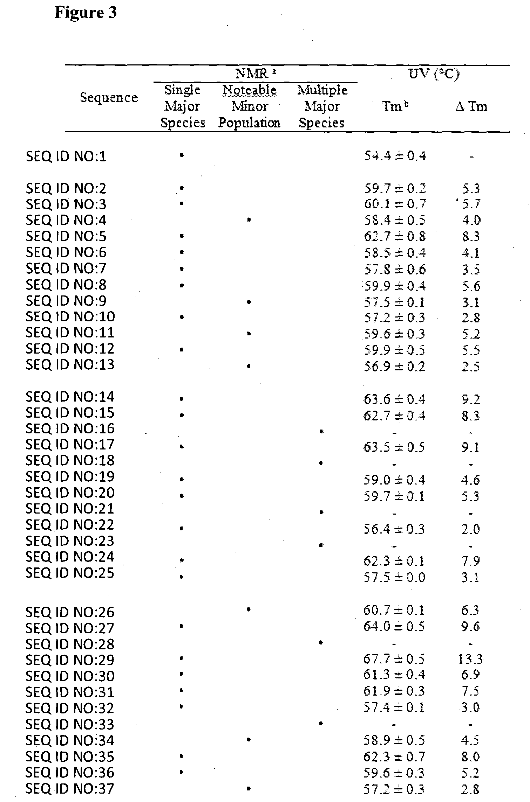

**Figure 3** shows the biophysical characterization of the indicated nucleic acid molecules.
**Figure 4** shows the design of highly stable G-quadruplex nanoparticles containing multiple N2-modifications: (A) Illustrative model of the "Ball-with-Hair" G-quadruplex nanoparticle formed by the N2-Ball with Hair sequence containing eight ^{Hex}G modifications (B-C) NMR and CD spectra of select nanoparticles. Each imino proton resonances in (B) is indicated with a dot (•). (D) Melting curves of designed sequences observed by CD at 290 nm in "low salt" (1 mM KPi and 1 mM KCl) conditions.

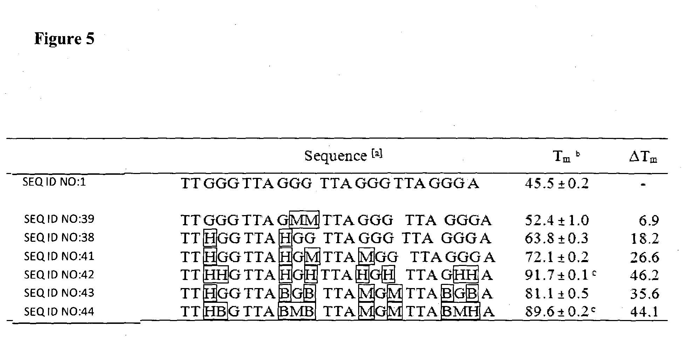

**Figure 5** shows the thermal stability (°C) of the indicated nucleic acid molecules containing multiple N2-substitutions under "low salt" conditions. The position of N2-modified guanines is denoted by a box with the following notation: "M" for N2-methyl-guanine; "H" for N2-6-amino-hexyl-guanine; "B" for N2-benzyl-guanine. [b] indicates that the nucleic acid molecule does not demonstrate a smooth melting transition.

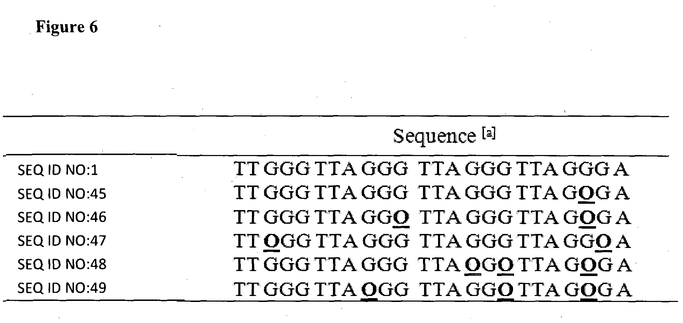

**Figure 6** shows nucleic acid molecules containing N2-modifications that comprise biotin. The position of N2-modified guanines is denoted by a box with the following notation: "O" for N2-C₁₂-Biotin-2'-deoxy guanosine-amidite.

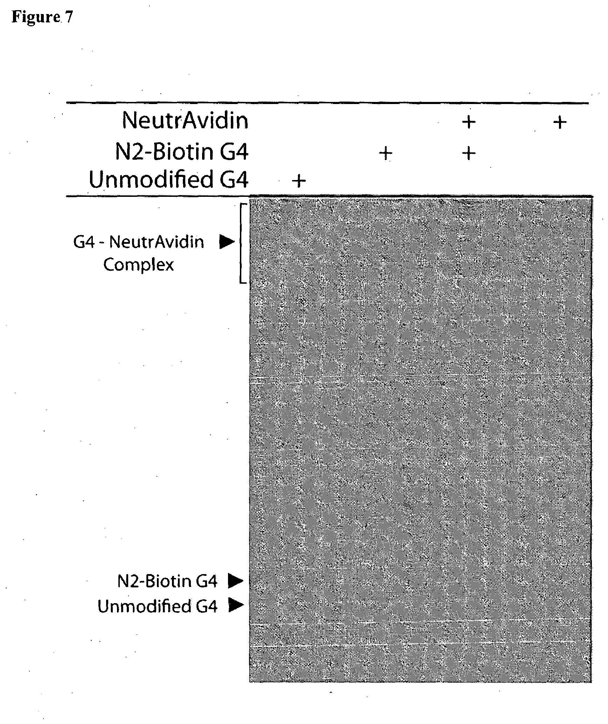

**Figure 7** shows applications of N2-modified G-quadruplex nanoparticles. Gel electrophoresis demonstrating the binding of biotin-modified G-quadruplex to NeutrAvidin protein. Gels were visualized with Bio-Rad Fast Blast DNA stain.

### DETAILED DESCRIPTION OF THE INVENTION

The present inventors surprisingly found that nucleic acid nanoparticles comprising at least one G-quadruplex structure, as defined in the appended claim 1,stabilize the nanoparticle and allow the attachment of additional chemical groups, such as pharmaceutically active compounds, to functionalize the nanoparticle. Furthermore, nucleic acid molecules containing at least one guanine base comprising a modification in the N2-position can be used as molecular sensors to detect different biological structures, such as G-quadruplexes.

Thus, in a first aspect, the present invention is directed to a nucleic acid nanoparticle comprising at least one G-quadruplex structure containing more than 2 G-tetrad layers wherein at least two guanine bases comprise a modification in the N2-position that stabilizes the nucleic acid nanoparticle, wherein the G-quadruplex structure comprises (a) a nucleic acid molecule comprising the nucleic acid sequence (g)w(n)a(g)x(n)b(g)y(n)c(g)z wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)z wherein z is an integer of at least 2; or (c) two nucleic acid molecules wherein each of said molecules comprises a sequence of (g)y(n)b(g)z wherein b is an integer of at least 0, y and z are independently of each other integers of at least 0, and the sum of integers y and z is at least 4; and wherein each modification is independently 6-aminohexyl or benzyl.

In a second aspect, the present invention relates to a nucleic acid nanoparticle as described herein for use as a medicament.

In a further aspect, the present invention relates to the use of a nucleic acid molecule as a molecular sensor, wherein said nucleic molecule comprises (a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)_{z} wherein z is an integer of at least 2; or (c) two nucleic acid molecules wherein each of said molecules comprises a sequence of (g)_{y}(n)_{b}(g)_{z} wherein b is an integer of at least 0, y and z are independently of each other integers of at least 0, and the sum of integers y and z is at least 4, wherein the nucleic acid molecule comprises at least 2 and preferably up to 10 N2-modified guanine bases, and wherein each modification is independently 6-aminohexyl or benzyl.

The G-quadruplex structure comprises (a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)_{z} wherein z is an integer of at least 2; or (c) two nucleic acid molecules wherein each of said molecules comprises a sequence of (g)_{y}(n)_{b}(g)_{z} wherein b is an integer of at least 0, y and z are independently of each other integers of at least 0, and the sum of integers y and z is at least 4. In various embodiments, the nucleic acid molecule comprises the nucleic acid sequence (g)_{w}(n)ₐ(g)ₓ(n)_{b}(g)_{y}(n)_{c}(g)_{z} wherein w,x,y,z are independently of each other integers of at least 3 and a,b,c are independently of each other integers of at least 1. In certain embodiments, the at least one N2-modified guanine base is located in one of the (g) sequence elements and not in the (n) sequence elements. In other certain embodiments, the G-quadruplex structure comprises a nucleic acid molecule having the nucleic acid sequence (n)₂-(g)₃-(n)₃-(g)₃-(n)₃-(g)₃-(n)₃-(g)₃-(n).

In various embodiments, the G-quadruplex structure comprises at least two N2-modified guanine bases. In various embodiments, the G-quadruplex structure comprises up to four N2-modified guanine bases. In various other embodiments, the G-quadruplex structure comprises up to 10 N2-modified guanine bases. In various other embodiments, all guanine bases are N2-modified.

The modification is independently 6-amino-hexyl or benzyl.

In various embodiments, the modification comprises a pharmaceutically active compound.

In various embodiments, the G-quadruplex structure comprises one nucleic acid molecule. In various other embodiments, the G-quadruplex structure comprises at least two nucleic acid molecules, such as 2, 3, 4, 5, 6, 7, 8, 9, ... nucleic acid molecules.

In various embodiments, the G-quadruplex structure has a diameter of 2 to 3 nm. In certain embodiments, the G-quadruplex structure comprises a nucleic acid molecule having a tag at its 3' and/or 5' end.

In various embodiments, a given guanine base comprises only one modification in its N2-postion. In various embodiments, the nucleic acid nanoparticle comprises only one G-quadruplex structure.

The terms "nucleic acid molecule", "nucleic acid sequence" or "oligonucleotide", as used herein, relate to any nucleic acid molecule in any possible configuration, including single-stranded, double-stranded, triple-stranded, quadruple stranded configurations or a combination thereof. Isolated oligonucleotides include for instance DNA molecules, RNA molecules and analogues of DNA or RNA comprising modified backbones, internucleotide linkages, sugars or bases. DNA or RNA may be of genomic or synthetic origin. Such nucleic acids include but are not limited to mRNA, cRNA, synthetic RNA, genomic DNA, cDNA, synthetic DNA and DNA/RNA hybrid. As described above, oligonucleotides are either synthetic constructs or nucleic acids separated from other cellular components with which it may naturally occur including cellular debris or are synthesized using known methods. The resulting nucleic acids are preferably 70, 80 or 90% pure, preferably at least 95 or 98% pure nucleic acid containing less than 30% contaminants, preferably less than 20 or 10% and most preferably less than 5 or 2% contaminants that cannot be identified as the nucleic acid as described herein. In preferred embodiments, the nucleic acid molecules of the invention comprise the sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8. In other certain embodiments, the G-quadruplex structure comprises a nucleic acid molecule having the nucleic acid sequence (g)_{w}(n)ₐ(g)ₓ(n)_{b}(g)_{y}(n)_{c}(g)_{z} wherein w,x,y,z are independently of each other integers of at least 3 and a,b,c are independently of each other integers of at least 1. In still other certain embodiments, the G-quadruplex structure comprises a nucleic acid molecule having the nucleic acid sequence (n)₂-(g)₃-(n)₃-(g)₃-(n)₃-(g)₃-(n)₃-(g)₃-(n). The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

Various nucleotide analogues are known and can be incorporated as part of, or replaced in its entirety, the isolated oligonucleotide of the present invention. A nucleotide analogue as defined herein is a nucleotide modified at the backbone, internucleotide linkage, sugar or base moiety. Modifications at the backbone or internucleotide linkage moiety include peptide nucleic acid (PNA) and substitution of the phosphate group by phosphorothioate. Modifications at the sugar moiety include locked nucleic acid (LNA) and substitution of the 2'-OH group. Modifications of the base moiety include alterations of A, T/U, G and C. Modifications of these different moieties can be applied on the same nucleotide in concert. Incorporation of nucleotide analogues within the isolated oligonucleotide can lead to improved nuclease resistance. Preferably, nucleic acid molecules of the present invention have at least one modification at N2-position of a given guanine base.

The term "guanine" or "guanine base", as used herein, relates to the base as shown in the formula below: 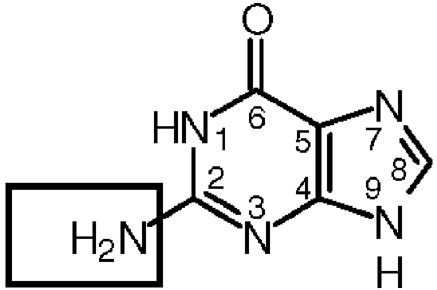 The term "N2-position", as used herein, relates to the amino group that is attached to the carbon at position 2 of guanine. In the above formula, said amino group is highlighted by the framed box.

The term "n", as used herein, relates to a nucleotide having a base that is selected from the group consisting of adenine, guanine, cytosine, uracil, and thymine.

"At least one", as used herein, relates to one or more, in particular 1, 2, 3, 4, 5, 6, 7, 8, 9, 10 or more.

The term "sequence element", as used herein, relates to the primary nucleotide sequence of nucleic acid molecules.

The term "G-quadruplex" refers to a four-stranded helical nucleic acid structure comprising multiple stacked G-tetrads, each of which consists of four guanine bases that associate in a cyclical manner through Hoogsteen hydrogen bonds and may be further stabilized through coordination to a cation in the center. The body of stacked G-tetrads, comprising a total of 2 or more layers, is collectively referred to as the G-tetrad core. Each of the four guanine columns constituting the G-tetrad core can arise from a single (continuous column) or two (discontinuous column) separate guanine stretch/es. The term "multimolecular" as used herein refers to a G-tetrad core that is formed by at least two separate oligonucleotide strands, each of which comprises at least one G-rich segment or partial G-rich segment.

The term "nanoparticle", as used herein, relates to a particle that is defined as a small object that behaves as a whole unit with respect to its transport and properties. In general, particles are classified according to their diameter. Nanoparticles are between 1 and 100 nanometers in size. In preferred embodiments, the G-quadruplex structure of the present invention has a diameter of 2 to 3 nm.

The term "modification" as used herein refers to carbon-containing moieties. These moieties can be linear or branched, substituted or unsubstituted, and are preferably derived from hydrocarbons, typically by substitution of one or more hydrogen or carbon atoms by other atoms, such as oxygen, nitrogen, sulfur, phosphorous, or functional groups that contain oxygen, nitrogen, sulfur, phosphorous. The organic moiety can comprise any number of carbon atoms, for example up to 5000 or more (typically in case of polymeric moieties), but preferably it is a low molecular weight organic moiety with up to 100, or more preferably up to 40 carbon atoms and, optimally, a molecular weight M_{w} of 1000 or less.

The organic moiety can be a linear or branched, substituted or unsubstituted C₁-Cₓ alkyl; linear or branched, substituted or unsubstituted C₂-Cₓ alkenyl; linear or branched, substituted or unsubstituted C₂-Cₓ alkinyl; linear or branched, substituted or unsubstituted Ci-Cₓ alkoxy; substituted or unsubstituted C₃-Cₓ cycloalkyl; substituted or unsubstituted C₃-Cₓ cycloalkenyl; substituted or unsubstituted C₆-Cₓ aryl; and substituted or unsubstituted C₃-Cₓ heteroaryl; with x being any integer of 2 or more, preferably up to 50, more preferably up to 30.

The modification can be a linear or branched, substituted or unsubstituted alkyl with 1 to 40 carbon atoms; linear or branched, substituted or unsubstituted alkenyl with 3 to 40 carbon atoms; linear or branched, substituted or unsubstituted alkoxy with 1 to 40 carbon atoms, substituted or unsubstituted cycloalkyl with 5 to 40 carbon atoms; substituted or unsubstituted cycloalkenyl with 5 to 40 carbon atoms; substituted or unsubstituted aryl with 5 to 40 carbon atoms; and substituted or unsubstituted heteroaryl with 5 to 40 carbon atoms.

The modification can be a linear or branched, substituted or unsubstituted alkyl with 1 to 20 carbon atoms; linear or branched, substituted or unsubstituted alkenyl with 3 to 20 carbon atoms; linear or branched, substituted or unsubstituted alkoxy with 1 to 20 carbon atoms, substituted or unsubstituted cycloalkyl with 5 to 20 carbon atoms; substituted or unsubstituted cycloalkenyl with 5 to 20 carbon atoms; substituted or unsubstituted aryl with 5 to 14 carbon atoms; and substituted or unsubstituted heteroaryl with 5 to 14 carbon atoms.

The modification can also be a combination of any of the above-defined groups, including but not limited to alkylaryl, arylalkyl, alkylheteroaryl and the like, to name only a few, all of which may be substituted or unsubstituted. According to the present invention, the modification is independently 6-aminohexyl or benzyl.

The term "substituted", as used herein, in relation to the above moieties refers to a substituent other than hydrogen. Such a substituent is preferably selected from the group consisting of halogen, -OH, -OOH, -NH₂, -NO₂, -ONO₂, -CHO, -CN, -CNOH, -COOH, -SH, -OSH, -CSSH, -SCN, -SO₂OH, -CONH₂, -NH-NH₂, -NC, -CSH -OR, -NRR', -C(O)R, - C(O)OR, -(CO)NRR', -NR'C(O)R, -OC(O)R, aryl with 5 to 20 carbon atoms, cycloalk(en)yl with 3 to 20 carbon atoms, 3- to 8-membered heterocycloalk(en)yl, and 5- to 20-membered heteroaryl, wherein R and R' are independently selected from hydrogen, alkyl with 1 to 10 carbon atoms, alkenyl with 2 to 10 carbon atoms, alkynyl with 2 to 10 carbon atoms, aryl with 5 to 14 carbon atoms, cycloalk(en)yl with 3 to 20 carbon atoms, 5- to 14- membered heteroaryl, comprising 1 to 4 heteroatoms selected from nitrogen, oxygen, and sulfur, and 5-to 14- membered heterocycloalk(en)yl, comprising 1 to 4 heteroatoms selected from nitrogen, oxygen, and sulfur. Any of these substituents may again be substituted, it is however preferred that these substituents are unsubstituted.

The term "alkyl", as used herein, relates to an alkane missing one hydrogen. An acyclic alkyl has the general formula CₙH₂ₙ₊₁.

Akenyl and alkynyl comprise at least one carbon-carbon double bonds or triple bonds, respectively, and are otherwise defined as alkyl above.

Cycloalkyl refers to a non-aromatic carbocyclic moiety, such as cyclopentanyl, cyclohexanyl and the like.

Cycloalkenyl refers to non-aromatic carbocyclic compounds that comprise at least one C-C double bond.

Similarly, heterocycloalk(en)yl relates to cycloalk(en)yl groups wherein 1 or more ring carbon atoms are replaced by heteroatoms, preferably selected from nitrogen, oxygen, and sulfur.

Aryl relates to an aromatic ring that is preferably monocyclic or consists of condensed aromatic rings. Preferred aryl substituents are moieties with 6 to 14 carbon atoms, such as phenyl, naphthyl, anthracenyl and phenanthrenyl.

Heteroaryl refers to aromatic moieties that correspond to the respective aryl moiety wherein one or more ring carbon atoms have been replaced by heteroatoms, such as nitrogen, oxygen, and sulfur.

All of the afore-mentioned groups can be substituted or unsubstituted. When substituted the substituent can be selected from the above list of substituents.

The term "N2-modified C₁₂-alkyl dithiolane" or "N2-C₁₂-alkyl dithiolane guanosine", as interchangeably used herein, relates to N2-modified guanine bases having the following formula:

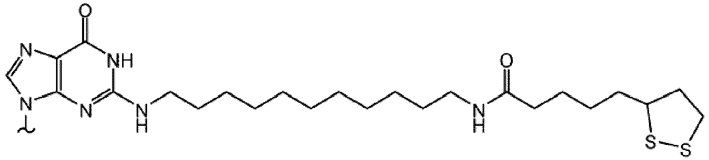

The term "N2-modified C₁₂-biotin" or "N2-C₁₂-biotin guanosine", as interchangeably used herein, relates to N2-modified guanine bases having the following formula:

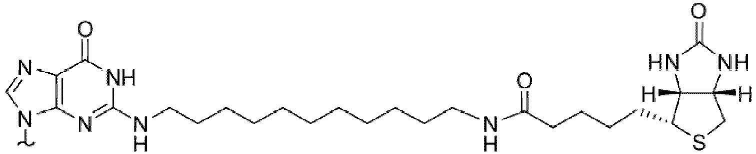

The term "3'end" or "3'hydroxyl end", as interchangeably used herein, relates to the termination at the hydroxyl group of the third carbon in the sugar-ring of a nucleic acid molecule, and is also known as the tail end. The term "5'end" or "5'phosphate end", as interchangeably used herein, designates the end of the DNA or RNA strand that has a phosphate group at the fifth carbon in the sugar-ring of the deoxyribose or ribose at its terminus.

"Pharmaceutically active compound", as used herein, relates to a substance in a pharmaceutical drug or a pesticide that is biologically active. Such compounds encompass a broad chemical variety and relate to molecules of different classes such as small molecules according to Lipinski's rule of five, proteins, nucleotides, lipids, sugars, and derivatives thereof.

The term "medicament", as used herein, relates to any chemical substance formulated or compounded as single active ingredient or in combination with other pharmacologically active substances. The formulated or compounded composition may comprise, besides the pharmaceutically active compound, a pharmaceutically acceptable carrier.

"Tag", as used herein, relates to a group of atoms or a molecule that is attached covalently to a nucleic acid sequence or another biological molecule for the purpose of detection by an appropriate detection system. The term "tagged nucleic acid nanoparticle" refers to a nucleic acid nanoparticle to which a tag has been covalently attached. The term "tag" and "label" may be used interchangeably.

The term "providing a nucleic acid nanoparticle", as used herein, relates to a given nucleic acid nanoparticle in any form. Thus, said nanoparticle may be solved in a specific solvent, for example water or alcohol, or appears in lyophilized form.

The term "molecular sensor", as used herein, relates to nucleic molecules comprising (a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8; (b) four nucleic acid molecules wherein each of said molecules comprises a sequence of (g)_{z} wherein z is an integer of at least 2; or (c) two nucleic acid molecules wherein each of said molecules comprises a sequence of (g)_{y}(n)_{b}(g)_{z} wherein b is an integer of at least 0, y and z are independently of each other integers of at least 0, and the sum of integers y and z is at least 4. Such molecular sensor may detect biological structures by binding to these structures or by being incorporated into said structures. For example, the nucleic acid molecule of the invention comprising the N2-modification may form a G-quadruplex structure together with other nucleic acid molecules. Subsequently, this G-quadruplex structure may be detected using detectable groups incorporated in the modification of the N2-position or that are attached at the 3' and/or 5' end of said nucleic acid molecule. Optionally, the nucleic acid molecule of the invention comprising the N2-modification may form a G-quadruplex structure on its own or may form such structure together with other nucleic acid molecules and the molecule forming the G-quadruplex may attach to a given biological structure. The detection of said structure may be done by detecting detectable groups within the N2-modification or by detecting detectable groups that are attached at 3' and/or 5' end of the nucleic acid molecule of the invention. The detectable group can be detected by several different techniques known in the art. Such techniques may include, but are not limited to fluorescence assay, immunoassay, mass spectrometry, chromatography, Western Blot, or gel electrophoresis.

In some embodiments, the immunoassay may be, but is not limited to an Enzyme-linked Immunosorbent Assay (ELISA), Western blot, agglutination test, biotin/avidin type assays, radioimmunoassays, immunoelectrophoresis and immunoprecipitation. The reactions generally include revealing labels such as fluorescent, chemiluminescent, radioactive, enzymatic labels or dye molecules, or other methods for detecting the formation of a complex. These and further immunoassays are well known in the art (David Wild (Ed.): The Immunoassay Handbook. 3rd ed. Elsevier Science Publishing Company, Amsterdam 2005).

The nucleic acid molecules of the invention may be synthesized synthetically. One specific method for such synthesis of oligonucleotides includes the use of nucleoside phosphoramidites. However, it is also possible that the nucleic acid molecules of the invention that contain the N2-position modification are naturally occurring molecules that are isolated and chemically modified. Another approach to synthesize the nucleic acid molecules of the invention is to utilize polymerase chain reaction (PCR) using polymerases that tolerate nucleosides having modified bases compared to the naturally occurring bases A, T, U, G and C.

The produced nucleic acid molecules are verified by sequencing. Sequencing of the nucleic acid molecules can be done by the chain termination method, Sanger sequencing or Maxam-Gilbert sequencing or any other technique known in the art. Alternatively, high-throughput sequencing, like pyrosequencing, SOLiD sequencing or DNA nanoball sequencing, is used to determine the sequence of the nucleic acid molecules of the present invention (Alphey, L. (1997) DNA Sequencing: From Experimental Methods to Bioinformatics, 1st Ed., Bios Scientific Pub Ltd., Oxford, UK). Although characterization can be done by sequencing, the nucleic acid molecules of the invention can also be characterized by recording their mass spectrum. Several mass spectrometry methods are described in the art and include, but are not limited to electrospray mass spectrometry (ES MS) and matrix-assisted laser desorption/ionization time-of-flight mass spectrometry (MALDI-TOF). Detailed protocols to characterize nucleic acid molecules with these methods are well-known in the art.

The term "administration", as used herein refers, to the delivery of an agent to a subject. Administration of the nucleic acid molecule described herein can be done by any appropriate route including, for example, oral, mucosal (e.g., nasal, sublingual, vaginal, rectal, etc), topical, transdermal, parenteral, etc. In some embodiments, the administration is non-invasive (e.g., is not parenteral).

### EXAMPLES

### Materials and Methods

### Synthesis and sample preparation

DNA samples were synthesized on an Applied Biosystems 394 DNA Synthesizer (Foster City, CA U.S.A.) and purified following Glen Research protocols. The modified phosphoramidite N2-6-amino-hexyl-guanine was purchased from Glen Research (Sterling, VA U.S.A.). Modified phosphoramidites N2-methyl-guanine and N2-benzyl-guanine were purchased from Berry & Associates (Dexter, MI U.S.A.). N2-modified C₁₂-biotin guanosine and N2-modified C₁₂-alkyl dithiolane guanosine phosphormadite was purchased from Chemgenes. Purified DNA samples were dialyzed against H2O, ~30 mM KCl, and H2O before being dried and dissolved in solution containing KCl, potassium phosphate (KPi) buffer (pH 7), 20 µM 4,4-dimethyl-4-silapentane-1-sulfonic acid used as a chemical shift reference in NMR, and containing 10% D2O. Concentrations of KPi and KCl varied to generate varying salt conditions, including "low salt" (1 mM KPi + 1 mM KCl), "moderate salt" (10 mM KPi + 10 mM KCl) and "physiological Salt" (10 mM KPi + 130 mM KCl).

### NMR spectroscopy

Spectra were obtained on a 600 MHz Bruker spectrometer (Billerica, MA U.S.A.) at a temperature of 25°C using a jump-and-return type water suppression pulse sequence. 39 samples were annealed prior to recording of NMR spectra by heating to 100°C for 2 minutes and allowed to cool slowly at room temperature. Chemical shifts were internally referenced with D.S.S.

### CD spectroscopy

Spectra were recorded on a JASCO-815 spectropolarimeter (Tokyo, Japan). Samples contained a DNA strand concentration of 4-5 µM. Spectra were recorded at 20°C in the range of 220-320 nm and averaged over 10 scans. Spectra were normalized through subtracting by the CD absorbance at 320 nm and dividing by concentration of the sample determined by concurrent UV absorption measurements. Samples were annealed prior to recording of CD spectra by heating to 100°C and allowed to cool slowly at room temperature.

### CD melting experiments

CD melting experiments were performed for the unmodified sequence and for sequences containing multiple N2-modifications. Samples contained 4-5 µM DNA by strand concentration in 1 mM KCl and 1 mM KPi. Samples were heated to 95°C (98°C for N2-Ball-with-Hair sample) and cooled to 15°C at a rate of 0.2°C/min. Samples were then heated in a similar manner. Data was recorded every 0.5°C at 290 nm and corrected by subtraction at 320 nm. Normalized melting curves are used to determine the melting temperature (Tm) of modified sequences, which is taken as an average of the heating and cooling curves. Hysteresis in a heating/cooling cycle was observed to be below 2°C for all samples.

### UV melting experiments

Melting experiments were performed on a JASCO V-650 UV-visible spectrophotometer (Tokyo, Japan). Samples containing ~5 µM DNA strand concentration were first heated to 83°C and cooled to 14°C at a rate of 0.2 °C/min. The absorbance was sampled every 0.5°C at 295 nm and 320 nm. At the end of the cooling cycle, samples were held for 10 minutes before being heated back to 83°C at the same rate. Melting curves were normalized first by subtraction at 320 nm and then further processed to create fraction folded curves. Melting temperatures (Tₘ) reported represent an average over heating and cooling cycles. Hysteresis of < 2°C (generally < 1.5°C) was observed for all sequences.

### Modeling

Models of Hex-3 and N2-Ball-with-Hair structures were built based on a NMR model of the unmodified (3+1) sequence (PDB ID 2GKU). Guanine bases containing N2-6-amino-hexyl modifications in a straight orientation were fit to guanine bases within the NMR structure. The dihedral angles around the connection of the guanine base to the N2-6-amino-hexyl functional group (Guanine(C2)-Guanine(N2)-Hex(C1)-Hex(C2)) were then manually varied to project the N2-6-amino-hexyl into the groove. Illustrative models were visualized in Pymol.

### Gel Electrophoresis

Native PAGE was performed in 10% polyacrylamide gel with a running buffer containing 10mM KCl and 10mM KPi to promote G-quadruplex formation. Gels were visualized with Bio-Rad Fast Blast DNA stain. Samples contained 10uM DNA/Neutravidin with K+ concentrations of ~ 20mM.

### Example 1: G-quadruplex nanoparticle formation of nucleic acid molecules containing single N2-guanine modifications

To functionalize a G-quadruplex scaffold with N2-modified guanine bases, a G-quadruplex structure formed from the sequence d[T₂(G₃T₂A)₃G₃A] was systematically substituted with N2-methyl-guanine (^{Met}G), N2-6-amino-hexyl-guanine (^{Hex}G), and N2-benzyl-guanine (^{Ben}G) (Figure 1). This sequence has been previously shown to form a G-quadruplex that demonstrates a complex (3+1) conformation with 3 strands oriented in one direction and one pointing in the opposite direction (Luu, KN et al. (2006) J Am Chem Soc 128, 30, pages 9963-9970). The orientation of these strands gives rise to grooves that vary greatly in their dimensions (Figure IB). These strands are connected by a fold-back propeller loop and two edge-wise loops. The core of the molecules is characterized by 3 stacked guanine tetrads, which contain bases that adopt both syn and anti glycosidic conformations. These diverse structural features provide an ideal test-scaffold for the functionalization of G-quadruplex nanoparticles through N2-modifications. The N2-modifications tested in this study were chosen as they vary greatly in complexity and shape. They include small (^{Met}G), bulky aromatic (^{Ben}G) and long chain-like (^{Hex}G) residues. The ability of modified nucleic acid molecules to form G-quadruplex nanoparticles was evaluated by monitoring the imino proton region of ¹H NMR spectra, a region characteristic of G-quadruplex formation. Imino proton spectra may be used to quickly evaluate the number of species in solution and similarity between spectra is a strong indicator that a similar conformation has been adopted by modified sequences. G-quadruplex formation was also monitored with CD spectroscopy, which provides spectral patterns characteristic of specific G-quadruplex folding topologies.

The systematic study explored a total of 36 sequences containing single-position N2-substitutions. All modified sequences demonstrated G-quadruplex formation as determined by the presence of distinct peaks in the imino proton region of NMR spectra (Figure 3). Of these sequences, 28 were observed to self-assembled into a single species adopting the same (3+1) conformation formed by the parent sequence based on the similarity of NMR and CD spectra (Figure 2). This included 10 of 12 for ^{Met}G (SEQ ID Nos. 2-13), 8 of 12 for ^{Hex}G (SEQ ID Nos. 14-25), and 10 of 12 for ^{Ben}G (SEQ ID Nos. 26-37) (Figure 3). Classification by groove type reveals that 5, 6, 8, and 9 sequences out of the 9 single-position substitutions tested per groove maintained a (3+1) conformation when substituted into the Narrow, Propeller, Wide, and Medium grooves respectively. Select N2-modifications were also tested in a parallel propeller type G-quadruplex scaffold with the formation of G-quadruplex observed via imino proton NMR spectra, suggesting that N2-guanine modifications can be successfully incorporated into a variety of G-quadruplex structures.

The combination of NMR and CD data suggested that N2-modifications do not inhibit G-quadruplex formation, as all tested nucleic acid molecules adopted the G-quadruplex structure. As hypothesized, most N2-modified guanines substitutions were successfully introduced without disrupting the native (3+1) conformation. It is not surprising that modifications into the Medium and Wide grooves were less disruptive to the primary G-quadruplex conformation compared with modifications into Narrow or Propeller-loop-containing grooves. It is noted that N2-modified guanine substitutions into positions 5 and 16 adopted a mixture of conformations and are particularly not tolerated in the (3+1) hybrid G-quadruplex structure. Examination of the NMR-based structure formed by the unmodified nucleic acid molecule does not yield any obvious reasons why these positions are not tolerated. It is important to note that multiple forms in NMR spectra can arise from the destabilization of the (3+1) structure and/or from the stabilization of an alternative G-quadruplex structure.

### Example 2: G-quadruplexes containing one N2-modified guanine display enhanced thermal stability

It was investigated how N2-guanine modifications affect the thermal stability of G-quadruplex nanoparticles in a "moderate salt" environment (10mM KPi + 10mM KCl). The stability of all sequences that maintained the native (3+1) G-quadruplex structure without substantial disruption upon modification was examined. Thermal denaturing studies were performed to determine the quantitative melting temperature (Tₘ) of the unmodified and N2-modified nanoparticles by monitoring the UV absorption as a function of temperature. Remarkably, all sequences that maintained the original (3+1) conformation demonstrated enhanced thermal stability (Figure 3), with Δ Tₘ values observed in the range of +2.0 to +13.2°C. On average, the stabilization by N2-modified guanines can be ranked as ^{Ben}G > ^{Hex}G > ^{Met}G with average Δ Tₘ values of 6.6°C, 6.0°C, and 4.6°C, respectively. Further classification of Δ Tₘ values by groove type reveal average Δ Tₘ values of 8.1°C, 5.3°C, 4.9°C and 4.5°C for the Propeller, Medium, Narrow, and Wide grooves, respectively.

It is very surprising that modifications stabilize the (3+1) G-quadruplex in case the original conformation is adopted. The stabilizing effects of N2-modifications are position dependent as modifications to certain residues, 4 and 9 for example, are observed to be highly stabilizing while some positions, such as 17 and 23, demonstrate moderate Tₘ enhancement. It is also surprising that N2-modifications into the Propeller-loop-containing groove yielded some of the most stable structures. Conversely, substitutions into the Wide groove yielded some of the most modest stabilization. Considering this, it was hypothesized that the stabilizing properties of N2-modifications may be due to favorable interactions with neighboring structural elements or with the solvent. It is also important to note that modifications replacing one of the H2 protons with a bulkier residue are thought to effect rotation about the N2-position, as supported by the emergence of sharp peaks in the region of 9.0-10.0 ppm in NMR spectra of most modified sequences.

### Example 3: Stabilizing effects of N2-substitutions accumulate in multiple N2-substituted G-quadruplex nanoparticles

The stabilizing properties of single N2-guanine modifications for the G-quadruplex scaffold led the inventors to pursue the design of a highly stabilized G-quadruplex nanoparticle. It was hypothesized that multiple N2-guanine substitutions would further enhance the stability of the molecular scaffold while simultaneously generating multiple sites to potentially functionalize the molecule. A number of sequences were synthesized containing a variety of N2-modifications in numerous positions based on the results of the single substitution studies (Figure 5).

SEQ ID NO:39 contains two ^{Met}G substitutions of guanines that neighbors within the wide groove, and tests the ability for a single groove to accommodate multiple modifications. SEQ ID NO:38 contains two ^{Hex}G substitutions into separate grooves in positions that were highly stabilizing in the single-substitutions studies. SEQ ID NO:41 was designed to contain a modification in each groove of the G-quadruplex nanoparticle. Also more ambitious designs were pursued, such as SEQ ID NO:42 containing eight ^{Hex}G modifications towards the design of a "Ball with Hair" type of nanoparticle (Figure 4A). Such a structure would be ideal for functionalization, with many long hexyl-chemistries capable of extending past the surface of the nanoparticle. SEQ ID Nos. 43 and 44 contain a variety of 8 and 10 modifications, respectively, aimed to maximize the thermal stability of these nanoparticle scaffolds.

The designed sequences were tested in a "low salt" solution of 1mM KPi + 1 mM KCl. Of the sequences tested with multiple modifications, SEQ ID Nos. 38, 39, 41 and 42 were determined to maintain the (3+1) conformation by analysis of ¹H NMR and CD spectra, which were highly similar to SEQ ID NO:1 (Figure 4). SEQ ID NO:43 revealed an NMR spectra that was slightly more crowded and a CD spectra with a notable decrease at 265nm. SEQ ID NO:44 demonstrated a similar NMR spectra as well as a characteristic (3+1) CD spectra that was slightly red-shifted compared to SEQ ID NO:1.

As desired, all of the sequences tested containing multiple N2-modifications displayed increasingly high thermal stability. SEQ ID Nos. 38, 39 and 41 displayed Δ Tₘ values of +6.9°C, +18.2°C and +26.6°C, respectively. Additionally, these changes in stability were roughly equivalent to the sum of stability changes observed for the individual single-position substitutions. The ball with hair design of SEQ ID NO:42 demonstrated the highest thermal stability, with a Δ Tₘ of +46.2°C and a melting temperature of 91.7°C in a low salt solution. SEQ ID Nos. 43 and 44 also demonstrated enhanced stabilities of +35.6 and an undetermined amount, respectively. SEQ ID NO:44 did not demonstrate a clear melting transition, but still had a substantial structure at 95°C.

The stability of the "Ball with Hair" structure formed by SEQ ID NO:42 under "physiological salt" conditions (10mM Kpi + 130 mM KCL) was similar to those experienced in a cellular environment. NMR spectra of SEQ ID NO:42 under physiological salt conditions matched the spectra recorded in low salt conditions. Temperature dependent CD spectra revealed the same signature CD spectra over varying temperature, with more than 80% signal intensity observed at 95°C. This data suggests that the engineered "ball with hair" G-quadruplex nanoparticle formed by SEQ ID NO:42 is an extremely stable structure with a melting temperature >95°C in physiological K⁺ conditions.

In order to investigate potential interaction capacity of N2-modified G-quadruplexes, a series of DNA containing an N2-dodecane amino-biotin linkage, termed N2-biotin series (Figure 6), was synthesized. As biotin is known to bind the StreptAvidin and related proteins with high affinity (Green, NM (1975) Adv Protein Chem 29, 85-133; Diamandis, EP and Christopoulos, TK (1991) Clin Chem 37, 625-636), the interaction of N2-biotin modified G-quadruplexes with Neutravidin protein was investigated using Gel electrophoresis (Figure 7). When N2-biotin modified G-quadruplex forming DNA was mixed with Neutravidin, multiple high molecular weight bands appear indicating that a DNA-Neutravdidin complex is formed.

The ability of multiple N2-modifications to increasingly stabilize the G-quadruplex without disrupting the folding topology demonstrates the great potential of the presently described tools. Modifications at the N2-position of guanine can be used to decorate the G-quadruplex scaffold in a ball-with-hair like fashion containing multiple sites for functionalization. Previous studies have shown that N2-modifications to G-quadruplexes can have beneficial properties on the inhibitory effects of aptamers (Koizumi, M et al. (1997) Bioorg Med Chem 5, 12, pages 2235-2243). Additionally, work by Koizumi et al. reported the addition of multiple small ^{Met}G substitutions into a tetramer G-quadruplex was observed to stabilize the scaffold.

The current application demonstrates the versatility of N2-modifications in a structurally diverse (3+1) G-quadruplex scaffold. The results indicate that single substitutions of N2-modified guanines are potent stabilizers of the G-quadruplex architecture and it was further demonstrated that it is possible to decorate this molecular scaffold with a variety of chemical groups, including long chain-like residues that can be used as a bridge to functionalize this molecular scaffold. Experimentally, a "ball with hair" type nanoparticle was designed through the addition of eight ^{Hex}G residues to the 3-layered G-quadruplex scaffold yielding in a remarkable increase of thermal stability, namely 46°C, without disrupting the conformation of the G-quadruplex nanoparticle.

## Claims

1. Nucleic acid nanoparticle comprising at least one G-quadruplex structure containing more than 2 G-tetrad layers wherein at least two guanine bases comprise a modification in the N2-position that stabilizes the nucleic acid nanoparticle,
wherein the G-quadruplex structure comprises
(a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8;
(b) four nucleic acid molecules wherein each of said molecules comprises a sequence of (g)_{z} wherein z is an integer of at least 2; or
(c) two nucleic acid molecules wherein each of said molecules comprises a sequence of (g)_{y}(n)_{b}(g)_{z}
wherein b is an integer of at least 0, y and z are independently of each other integers of at least 0, and the sum of integers y and z is at least 4; and
wherein each modification is independently 6-aminohexyl or benzyl.

2. The nucleic acid nanoparticle according to claim 1(a), wherein the G-quadruplex structure comprises the nucleic acid sequence (g)_{w}(n)ₐ(g)ₓ(n)_{b}(g)_{y}(n)_{c}(g)_{z} wherein w,x,y,z are independently of each other integers of at least 3, and a,b,c are independently of each other integers of at least 1.

3. The nucleic acid nanoparticle according to claim 2, wherein the G-quadruplex structure comprises a nucleic acid molecule having the nucleic acid sequence (n)2-(g)3-(n)3-(g)3-(n)3-(g)3-(n)3-(g)₃-(n) .

4. The nucleic acid nanoparticle according to claims 1 to 3, wherein the G-quadruplex structure comprises up to 4, 8 or up to 10 N2-modified guanine bases or wherein all guanine bases are N2 modified.

5. The nucleic acid nanoparticle according to claims 1 to 4 wherein the modification comprises a pharmaceutically active compound.

6. The nucleic acid nanoparticle according to claims 1 to 5 wherein the G-quadruplex structure has a diameter of 2 to 3 nm.

7. The nucleic acid nanoparticle according to claims 1 to 6 wherein the G-quadruplex structure comprises a nucleic acid molecule having a tag at its 3' and/or 5' end.

8. Nucleic acid nanoparticle according to claims 1 to 7 for use as a medicament.

9. Use of a nucleic acid molecule as a molecular sensor for detecting biological structures,
wherein said nucleic acid molecule comprises
(a) a nucleic acid molecule comprising the nucleic acid sequence (g)_{w}(n)ₐ(g)ₓ(n)_{b}(g)_{y}(n)_{c}(g)_{z} wherein w,x,y,z are independently of each other integers of at least 0, a,b,c are independently of each other integers of at least 0, and the sum of integers w,x,y,z is at least 8;
(b) four nucleic acid molecules wherein each of said molecules comprises a sequence of (g)_{z} wherein z is an integer of at least 2; or
(c) two nucleic acid molecules wherein each of said molecules comprises a sequence of (g)_{y}(n)_{b}(g)_{z}
wherein b is an integer of at least 0, y and z are independently of each other integers of at least 0, and the sum of integers y and z is at least 4,
wherein the nucleic acid molecule comprises at least 2 and preferably up to 10 N2-modified guanine bases, and wherein each modification is independently 6-aminohexyl or benzyl

10. Use according to claim 9, wherein said nucleic acid molecule detects a G-quadruplex structure.

11. Use according to claim 9(a), wherein the nucleic acid molecule has the nucleic acid sequence (n)2-(g)3-(n)3-(g)3-(n)3-(g)3-(n)3-(g)3-(n).

## Patentansprüche

1. Nukleinsäure-Nanopartikel, umfassend mindestens eine G-Quadruplex-Struktur, enthaltend mehr als 2 G-Tetrade-Schichten, wobei mindestens zwei Guaninbasen eine Modifikation in der N2-Position, die den Nukleinsäure-Nanopartikel stabilisiert, umfassen
wobei die G-Quadruplex-Struktur umfasst
(a) ein Nukleinsäuremolekül, umfassend die Nukleinsäuresequenz (g)_{w}(n)a(g)ₓ(n)_{b}(g)_{y}(n)c(g)_{z}, wobei w,x,y,z unabhängig voneinander ganze Zahlen von mindestens 0 sind, a,b,c unabhängig voneinander ganze Zahlen von mindestens 0 sind und die Summe der ganzen Zahlen w,x,y,z mindestens 8 beträgt;
(b) vier Nukleinsäuremoleküle, wobei jedes der Moleküle eine Sequenz (g)_{z} umfasst, wobei z eine ganze Zahl von mindestens 2 ist; oder
(c) zwei Nukleinsäuremoleküle, wobei jedes der Moleküle eine Sequenz (g)_{y}(n)_{b}(g)_{z} umfasst, wobei b eine ganze Zahl von mindestens 0 ist, y und z unabhängig voneinander ganze Zahlen von mindestens 0 sind und die Summe der ganzen Zahlen y und z mindestens 4 beträgt; und
wobei jede Modifikation unabhängig 6-Aminohexyl oder Benzyl ist.

2. Der Nukleinsäure-Nanopartikel gemäß Anspruch 1(a), wobei die G-Quadruplex-Struktur die Nukleinsäuresequenz (g)_{w}(n)ₐ(g)ₓ(n)_{b}(g)_{y}(n)_{c}(g)_{z} umfasst, wobei w,x,y,z unabhängig voneinander ganze Zahlen von mindestens 3 sind und a,b,c unabhängig voneinander ganze Zahlen von mindestens 1 sind.

3. Der Nukleinsäure-Nanopartikel gemäß Anspruch 2, wobei die G-Quadruplex Struktur mindestens ein Nukleinsäuremolekül, das die Nukleinsäuresequen (n)2-(g)3-(n)3-(g)3-(n)3-(g)3-(n)3-(g)3-(n) aufweist, umfasst.

4. Der Nukleinsäure-Nanopartikel gemäß den Ansprüchen 1 bis 3, wobei die G-Quadruplex-Struktur bis zu 4, 8 oder bis zu 10 N2-modifizierte Guaninbasen umfasst oder wobei alle Guaninbasen N2-modifiziert sind.

5. Der Nukleinsäure-Nanopartikel gemäß den Ansprüchen 1 bis 4, wobei die Modifikation eine pharmazeutisch-aktive Verbindung umfasst.

6. Der Nukleinsäure-Nanopartikel gemäß den Ansprüchen 1 bis 5, wobei die G-Quadruplex-Struktur einen Durchmesser von 2 bis 3 nm aufweist.

7. Der Nukleinsäure-Nanopartikel gemäß den Ansprüchen 1 bis 6, wobei die G-Quadruplex-Struktur ein Nukleinsäuremolekül, das einen Tag an seinem 3' und/oder 5'-Ende aufweist, umfasst.

8. Nukleinsäure-Nanopartikel gemäß den Ansprüchen 1 bis 7 zur Verwendung als ein Medikament.

9. Verwendung eines Nukleinsäuremoleküls als einen molekularen Sensor zum Nachweisen biologischer Strukturen, wobei das Nukleinsäuremolekül umfasst
(a) ein Nukleinsäuremolekül, umfassend die Nukleinsäuresequenz (g)_{w}(n)a(g)ₓ(n)_{b}(g)_{y}(n)c(g)_{z}, wobei w,x,y,z unabhängig voneinander ganze Zahlen von mindestens 0 sind, a,b,c unabhängig voneinander ganze Zahlen von mindestens 0 sind und die Summe der ganzen Zahlen w,x,y,z mindestens 8 beträgt;
(b) vier Nukleinsäuremoleküle, wobei jedes der Moleküle eine Sequenz (g)_{z} umfasst, wobei z eine ganze Zahl von mindestens 2 ist; oder
(c) zwei Nukleinsäuremoleküle, wobei jedes der Moleküle eine Sequenz (g)_{y}(n)_{b}(g)_{z} umfasst, wobei b eine ganze Zahl von mindestens 0 ist, y und z unabhängig voneinander ganze Zahlen von mindestens 0 sind und die Summe der ganzen Zahlen y und z mindestens 4 beträgt;
wobei das Nukleinsäuremolekül mindestens 2 und bevorzugt bis zu 10 N2-modifizierte Guaninbasen umfasst, und wobei jede Modifikation unabhängig 6-Aminohexyl oder Benzyl ist.

10. Verwendung gemäß Anspruch 9, wobei das Nukleinsäuremolekül eine G-Quadruplex-Struktur nachweist.

11. Verwendung gemäß Anspruch 9(a), wobei das Nukleinsäuremolekül die Nukleinsäuresequenz (n)2-(g)3-(n)3-(g)3-(n)3-(g)3-(n)3-(g)3-(n) aufweist.

## Revendications

1. Nanoparticule d'acides nucléiques comprenant au moins une structure G-quadruplex comportant plus de deux couches de G-tétrades, dans laquelle au moins deux bases guanine comprennent une modification en position N2 qui stabilise la nanoparticule d'acides nucléiques, dans laquelle la structure G-quadruplex comprend
(a) une molécule d'acides nucléiques comprenant la séquence d'acides nucléiques (g)_{w}(n)ₐ(g)ₓ(n)_{b}(g)_{y}(n)_{c}(g)_{z}, dans laquelle w, x, y et z sont, indépendamment les uns des autres, des entiers supérieurs ou égaux à 0, a, b et c sont, indépendamment les uns des autres, des entiers supérieurs ou égaux à 0, et la somme des entiers w, x, y et z est supérieure ou égale à 8 ;
(b) quatre molécules d'acides nucléiques, dans laquelle chacune desdites molécules comprend une séquence (g)_{z}, dans laquelle z est un entier supérieur ou égal à 2 ; ou
(c) deux molécules d'acides nucléiques, dans laquelle chacune desdites molécules comprend une séquence (g)_{y}(n)_{b}(g)_{z}, dans laquelle b est un entier supérieur ou égal à 0, y et z sont, indépendamment l'un de l'autre, des entiers supérieurs ou égaux à 0, et la somme des entiers y et z est supérieure ou égale à 4 ; et
dans laquelle chaque modification est indépendamment un 6-aminohexyle ou un benzyle.

2. La nanoparticule d'acides nucléiques selon la revendication 1(a), dans laquelle la structure G-quadruplex comprend la séquence d'acides nucléiques (g)_{w}(n)a(g)ₓ(n)_{b}(g)_{y}(n)_{c}(g)_{z}, dans laquelle w, x, y et z sont, indépendamment les uns des autres, des entiers supérieurs ou égaux à 3, et a, b et c sont, indépendamment les uns des autres, des entiers supérieurs ou égaux à 1.

3. La nanoparticule d'acides nucléiques selon la revendication 2, dans laquelle la structure G-quadruplex comprend une molécule d'acides nucléiques comportant la séquence d'acides nucléiques (n)₂-(g)₃-(n)₃-(g)₃-(n)₃-(g)₃-(n)₃-(g)₃-(n).

4. La nanoparticule d'acides nucléiques selon les revendications 1 à 3, dans laquelle la structure G-quadruplex comprend jusqu'à 4, 8 ou 10 bases guanine modifiées en N2 ou dans laquelle toutes les bases guanine sont modifiées en N2.

5. La nanoparticule d'acides nucléiques selon les revendications 1 à 4, dans laquelle la modification comprend un composé pharmaceutiquement actif.

6. La nanoparticule d'acides nucléiques selon les revendications 1 à 5, dans laquelle la structure G-quadruplex a un diamètre de 2 à 3 nm.

7. La nanoparticule d'acides nucléiques selon les revendications 1 à 6, dans laquelle la structure G-quadruplex comprend une molécule d'acides nucléiques dont l'extrémité 3' et/ou 5' présente un marqueur.

8. La nanoparticule d'acides nucléiques selon les revendications 1 à 7, destinée à être utilisée comme médicament.

9. Utilisation d'une molécule d'acides nucléiques en tant que capteur moléculaire permettant de détecter des structures biologiques, dans laquelle ladite molécule d'acides nucléiques comprend
(a) une molécule d'acides nucléiques comprenant la séquence d'acides nucléiques (g)_{w}(n)ₐ(g)ₓ(n)_{b}(g)_{y}(n)_{c}(g)_{z}, dans laquelle w, x, y et z sont, indépendamment les uns des autres, des entiers supérieurs ou égaux à 0, a, b et c sont, indépendamment les uns des autres, des entiers supérieurs ou égaux à 0, et la somme des entiers w, x, y et z est supérieure ou égale à 8 ;
(b) quatre molécules d'acides nucléiques, dans laquelle chacune desdites molécules comprend une séquence (g)_{z}, dans laquelle z est un entier supérieur ou égal à 2 ; ou
(c) deux molécules d'acides nucléiques, dans laquelle chacune desdites molécules comprend une séquence (g)_{y}(n)_{b}(g)_{z}, dans laquelle b est un entier supérieur ou égal à 0, y et z sont, indépendamment l'un de l'autre, des entiers supérieurs ou égaux à 0, et la somme des entiers y et z est supérieure ou égale à 4,
dans laquelle la molécule d'acides nucléiques comprend au moins 2 et de préférence jusqu'à 10 bases guanine modifiées en N2, et dans laquelle chaque modification est indépendamment un 6-aminohexyle ou un benzyle.

10. Utilisation selon la revendication 9, dans laquelle ladite molécule d'acides nucléiques détecte une structure G-quadruplex.

11. Utilisation selon la revendication 9(a), dans laquelle la molécule d'acides nucléiques comporte la séquence d'acides nucléiques (n)₂-(g)₃-(n)₃-(g)₃-(n)₃-(g)₃-(n)₃-(g)₃-(n).
